# EUROPEAN PATENT SPECIFICATION

(11) **EP 1 686 177 B1**
(45) Date of publication and mention of the grant of the patent: **17.03.2010**
(21) Application number: 06001626.8
(22) Date of filing: 26.01.2006
(51) Int. Cl.: C12N 15/82, A01H 5/00, G01N 33/53, C12Q 1/68

(54) **Method for enhancing environmental stress resistance of plant using environmental stress associated gene**
Verfahren zur Erhöhung der Resistenz von Pflanzen gegenüber Umweltstressfaktoren unter Verwendung von einem mit Umweltstress verbundenem Gen
Procédé améliorant la résistance des plantes aux stress environnementaux en utilisant un gène associé avec le stress environmental

(30) Priority: 27.01.2005 KR 2005007534
(43) Date of publication of application: 02.08.2006
(73) Proprietor: Seoul National University Industry Foundation, Seoul 151-742 (KR)
(72) Inventor: Kim, Min-Kyun, School of Agricultural Biotechnology Seoul National Univ., Kwanak-gu Seoul 151-921 (KR); Jung, Jin-Wook, Gwanak-gu, Seoul 151-867 (KR)
(74) Representative: Bockhorni & Kollegen

(56) References cited:
- WO-A-02/16655
- WO-A-99/38977
- WO-A-02/079245
- WO-A-03/020936
- US-A1- 2003 131 386
- US-A1- 2004 034 888
- US-A1- 2004 045 049
- KASUGA MIE ET AL: "Improving plant drought, salt, and freezing tolerance by gene transfer of a single stress-inducible transcription factor" NATURE BIOTECHNOLOGY, NATURE PUB. CO, NEW YORK, NY, US, vol. 17, no. 3, March 1999 (1999-03), pages 287-291, XP002173128 ISSN: 1087-0156
- SAKUMA Y ET AL: "DNA-binding specificity of the ERF/AP2 domain of Arabidopsis DREBs, transcription factors involved in dehydration- and cold-inducible gene expression" BIOCHEMICAL AND BIOPHYSICAL RESEARCH COMMUNICATIONS, ACADEMIC PRESS INC. ORLANDO, FL, US, vol. 290, 2002, pages 998-1009, XP002982302 ISSN: 0006-291X
- OKAMURO J ET AL: "The AP2 domain of APETALA2 defines a large new family of DNA binding proteins in Arabidopsis" PROCEEDINGS OF THE NATIONAL ACADEMY OF SCIENCES OF USA, NATIONAL ACADEMY OF SCIENCE, WASHINGTON, DC, US, vol. 94, no. 13, June 1997 (1997-06), pages 7076-7081, XP002108957 ISSN: 0027-8424
- KIZIS D ET AL: "Role of AP2/EREBP transcription factors in gene regulation during abiotic stress" FEBS LETTERS, ELSEVIER, AMSTERDAM, NL, vol. 498, no. 2-3, 8 June 2001 (2001-06-08), pages 187-189, XP004597902 ISSN: 0014-5793
- LIU QIANG ET AL: "Two transcription factors, DREB1 and DREB2, with an EREBP/AP2 DNA binding domain separate two cellular signal transduction pathways in drought-and low-temperature-responsive gene expression, respectively, in Arabidopsis" PLANT CELL, AMERICAN SOCIETY OF PLANT PHYSIOLOGISTS, ROCKVILLE, MD, US, vol. 10, no. 8, August 1998 (1998-08), pages 1391-1406, XP002145075 ISSN: 1040-4651
- QIN J ET AL: "Isolation and characterization of an ERF-like gene from Gossypium barbadense" PLANT SCIENCE, LIMERICK, IE, vol. 167, no. 6, December 2004 (2004-12), pages 1383-1389, XP004572694 ISSN: 0168-9452
- ZHANG JI-YI ET AL: "Overexpression of WXP1, a putative Medicago truncatula AP2 domain-containing transcription factor gene, increases cuticular wax accumulation and enhances drought tolerance in transgenic alfalfa (Medicago sativa)" PLANT JOURNAL, vol. 42, no. 5, June 2005 (2005-06), pages 689-707, XP002378861 ISSN: 0960-7412
- VALLIYODAN ET AL: "Understanding regulatory networks and engineering for enhanced drought tolerance in plants" CURRENT OPINION IN PLANT BIOLOGY, QUADRANT SUBSCRIPTION SERVICES, GB, vol. 9, no. 2, April 2006 (2006-04), pages 189-195, XP005306212 ISSN: 1369-5266
- LIU ET AL: "The conserved Ala37 in the ERF/AP2 domain is essential for binding with the DRE element and the GCC box" FEBS LETTERS, ELSEVIER, AMSTERDAM, NL, vol. 580, no. 5, 20 February 2006 (2006-02-20), pages 1303-1308, XP005289531 ISSN: 0014-5793

## Description

### TECHNICAL FIELD

The present invention relates to a method for enhancing the environmental stress resistance of plants using an environmental stress resistance-controlling gene, wherein the environmental stress is paraquat or diquat.

### BACKGROUND ART

Environmental stress acts as a factor that suppresses the growth of plants and limits the production of crops in many important agricultural fields. A typical example of such environmental stress is drought stress. The drought stress causes osmotic imbalance and ionic imbalance in plant cells so as to suppress the growth and photosynthesis of plants. Meanwhile, plants have defense mechanisms against such drought stress so that, when subjected to an environment unsuitable for their growth, they will tend to control their configuration or physiological metabolism thereby adapt to the environment to survive. The drought resistance mechanisms in plants include abscisic acid (ABA)-dependent signaling pathways or ABA-independent signaling pathways (Finkelstein et al., Plant Cell 14, S15-S45, 2002; Himmelbach et al., Curr-Opin Plant Biol 6, 470-479, 2003; Kuhn and Schroeder, Curr Opin Plant Biol 6:463-469, 2003). The ABA is a typical drought-associated gene in plants and known to be associated also with the germination and vegetative growth and the opening and closing of guard cells in plants. When the concentration of ABA in guard cells increases, ions, such as potassium ions (K⁺) and chlorine ions (Cl⁻), will be released extracellularly, and intracellular calcium ions (Ca²⁺) will increase. Thus, the turgor pressure of plants will decrease to close pores and to inhibit transpiration, thus minimizing the loss of water from the plants.

However, if drought is lasting or its extent is large, the development, growth and crop yield of plants will be reduced, and lasting drought conditions will cause variations in plant metabolisms. These metabolic variations will ultimately lead to the death of plant cells.

For this reason, many studies to render plants resistant to the drought stress have been conducted. For example, PCT Patent Publication No. WO 2005/048693 discloses a method for producing plants having enhanced drought resistance by reducing or inhibiting the function of a cap binding protein gene in plants. Also, PCT Patent Publication No. WO 2004/058963 discloses a method for producing plants having enhanced drought resistance by transforming plants with an Oryza sativa-derived *OSISAPI* gene.

Meanwhile, herbicide, another important factor limiting the growth and development of plants, acts to directly suppress important plant enzymes or proteins necessary for the growth of plants so as to inhibit the growth of the plants or wither the plants. For example, herbicide glyphosate destroys plants by suppressing the activity of enzymes necessary for the synthesis of aromatic amino acids (Fillatti et al., Biotechnology, 5:726-730, 1987). Also, phosphinothricin suppresses the synthesis of glutamine, and atrazine suppresses photosynthetic mechanisms, and paraquat (1,1'-dimethyl-4,4'-bipyridylium dichloride) induces cell injury by producing a large amount and various kinds of active oxygen species in the presence of light.

In an attempt to enhance the resistance of plants to such herbicides, methods of transforming plants with herbicide-resistant genes isolated from microorganisms have mainly been used. For example, it was reported that potato, wheat, rice, corn, etc., were imparted with herbicide resistance by transforming the plants with herbicide-resistant genes of decomposing phosphinothricin herbicides, in which the genes were isolated from *Streptomyces hygroscopicus.*

Meanwhile, in recent years, there have been studies on defense mechanisms, which can induce a broader spectrum of resistance by overcoming shortcomings in that defense mechanisms against environmental stress, which have been studied until now, are species-specific or environment-specific.

### SUMMARY OF THE INVENTION

Accordingly, the present inventors have made studies to develop a method for enhancing the resistance of plants to various environmental stresses and as a result, found that, when an *AIA* gene having a known base sequence but unknown functions is introduced into plants, the *AIA* gene will have an activity of enhancing the resistance of plants to environmental stresses, such as herbicide, thereby completing the present invention.

Therefore, it is an object of the present invention to provide a method for enhancing the environmental stress resistance of plants using an environmental stress resistance-controlling gene, whose function has been newly found in the present invention.

To achieve the above object, in one aspect, the present invention provides A method for enhancing the environmental stress resistance of plants, consisting of introducing an AIA (ABA inducible AP2/ERF transcription factor) polynucleotide-comprising recombinant vector into the plants, wherein the polynucleotide encodes a polypeptide comprising an amino acid sequence of SEQ ID NO: 2, and wherein the environmental stress is paraquat or diquat.

In another aspect, the present invention provides a method for producing environmental stress-resistant plants, consisting of introducing an AIA (ABA inducible AP2/ERF transcription factor) polynucleotide-comprising recombinant vector into the plants, wherein the polynucleotide encodes a polypeptide comprising an amino acid sequence of SEQ ID NO: 2, wherein the environmental stress is a herbicide selected from the group consisting of paraquat or diquat.

Hereinafter, the present invention will be described in detail.

### DETAILED DESCRIPTION OF THE INVENTION

### Definition

Unless otherwise stated, all technical and scientific terms used herein have the same meanings as commonly understood by those skilled in the art. The following references provide one skilled in the art with general definitions of various terms and expressions used herein: Singleton et al., DICTIONARY OF MICROBIOLOGY ANDMOLECULAR BIOLOTY (2d ed. 1994); THE CAMBRIDGE DICTIONARY OF SCIENCE AND TECHNOLOGY (Walker ed., 1988); and Hale & Marham, THE HARPER COLLINS DICTIONARY OF BIOLOGY.

As used herein, the term "environmental stress" refers to an external factor that suppresses the growth or production of plants. Preferably, this term refers to stress caused by herbicide.

As used herein, the term "environmental stress resistance" refers to a characteristic in that a reduction in the growth or production of plants, caused by environmental stress, is inhibited or delayed.

As used herein, the term "polynucleotide" refers to a nucleic acid molecule comprising a plurality of polymerized nucleotides. The polynucleotide may be, for example, genomic DNA, RNA, cDNA, PCR product, cloned DNA, synthetic DNA or RNA. The polynucleotide preferably has a nucleotide sequence encoding a polypeptide (or protein) or its domain or fragment.

As used herein, the term "polypeptide" is used interchangeably with the terms "peptide" or "protein" and refers to, e.g., a polymer of amino acid residues.

As used herein, the term "overexpression" means the expression level of the inventive polynucleotide in plants, plant cells or plant tissues is higher than in that wild-type plants.

As used herein, the term "wild type" refers to a plant cell, seed, plant component, plant tissue, plant organ or whole plant, which has been genetically unmodified or untreated in an experimental sense.

As used herein, the term "expression vector" means plasmids, virus or other vehicles known in the art, that has been manipulated by inserting or introduction of the polynucleotide sequence of the present invention.

As used herein, the term "operably linked" may mean that the polynucleotide is linked to an expression control sequence in such a manner to enable expression of the polynucleotide when a suitable molecule is bound to the expression control sequence.

As used herein, the term "expression control sequence" means a DNA sequence that regulates the expression of the operably linked nucleic acid sequence in a certain host cell.

The present inventors have found for the first time that a known gene called *"RAP2.12* " having unknown functions, which belongs to the AP2/ERF family and is isolated from *Arabidopsis thaliana,* has a function of controlling the environmental stress resistance of plants. The present inventors newly named the *RAP2.12* gene *"AIA"* (ABA Inducible AP2/ERF transcription factor).

To identify the function of the *AIA* gene, the present inventors prepared the cDNA of the *AIA* gene by performing RT-PCR using total RNA isolated from *Arabidopsis thaliana* as a template and primers designed on the basis of a known base sequence (GenBank accession no: Atlg53910) (see Example 1). The base sequence and amino acid sequence of the *AIA* gene are shown in SEQ ID NO: 1 and SEQ ID NO: 2, respectively.

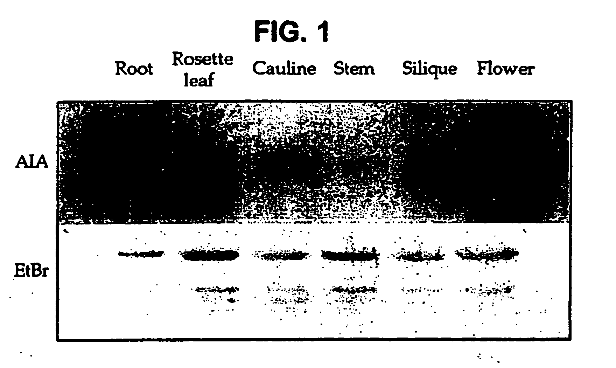

To examine the characteristic of the *AIA* gene, the present inventors analyzed the expression pattern of the gene in wild-type *Arabidopsis thaliana* using Northern blot and RT-PCR (see Example 1). As a result, it could be found that the gene was expressed in all the organs of wild-type *Arabidopsis thaliana,* and particularly, highly expressed in rosette leaves and roots (see FIG. 1). Also, the gene was expressed in both the leaves and roots of *Arabidopsis thaliana* at the early and later stages of germination. Thus, it was inferred that the gene would perform a specific function throughout the life cycle of plants while existing in most tissues of the plants.

Thus, to examine whether the gene has an activity of controlling the environmental stress resistance of plants, the present inventors conducted the following experiment.

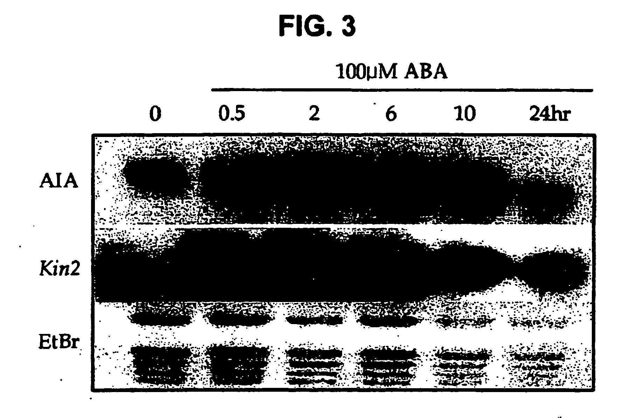

Based on the fact that most of drought resistance-associated genes are induced by treatment with plant hormone ABA, *Arabidopsis thaliana* was treated with various plant hormones, including ABA, and then analyzed for the expression of the *AIA* gene by Northern blot (see Example 3). As a result, it could be found that the gene was induced by treatment with ABA (see FIG. 3). On the other hand, treatment of plants with hormones, such as auxin, gibberellin, jasmonic acid and ethephon, had no great effect on the expression of the *AIA* gene. Thus, it was inferred that the *AIA* gene would perform a function of controlling the drought resistance of plants.

To prove this inference, the present inventors produced transgenic plants overexpressing the *AIA* gene (see Example 4) and homozygous mutants with inhibited expression of the *AIA* gene (see Example 5). The produced plants were analyzed for drought resistance (see Example 6).

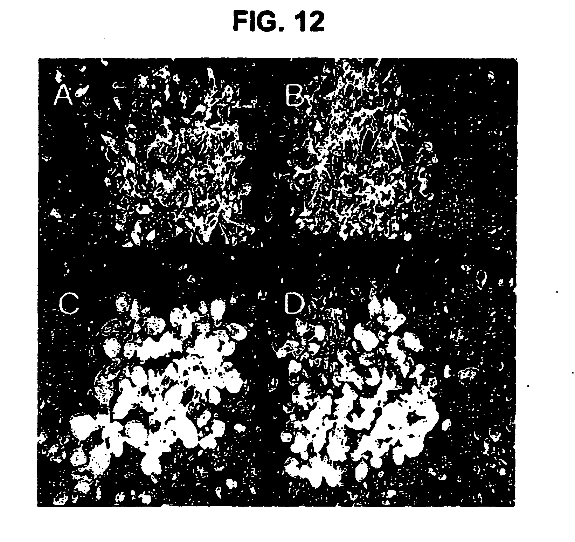

As a result, it was shown that the *AIA* gene-overexpressed transgenic plants mostly survived even in the presence of drought stress, whereas the wild-type plants and the mutants with inhibited expression of the *AIA* gene showed a death rate of more than 80% (see FIG. 12).

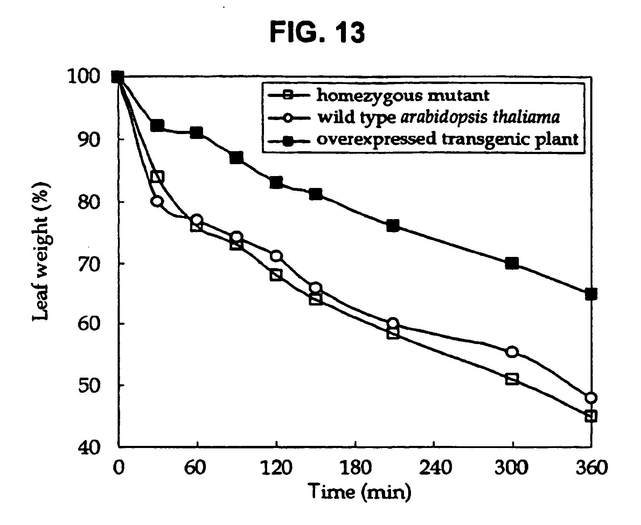

Furthermore, the *AIA* gene-overexpressed transgenic plants according to the present invention showed a reduction in the loss of water with the passage of time, compared to wild-type *Arabidopsis thaliana* and the mutants (see FIG. 13).

Accordingly, it could be found that the *AIA* gene has a function of enhancing the drought resistance of plants.

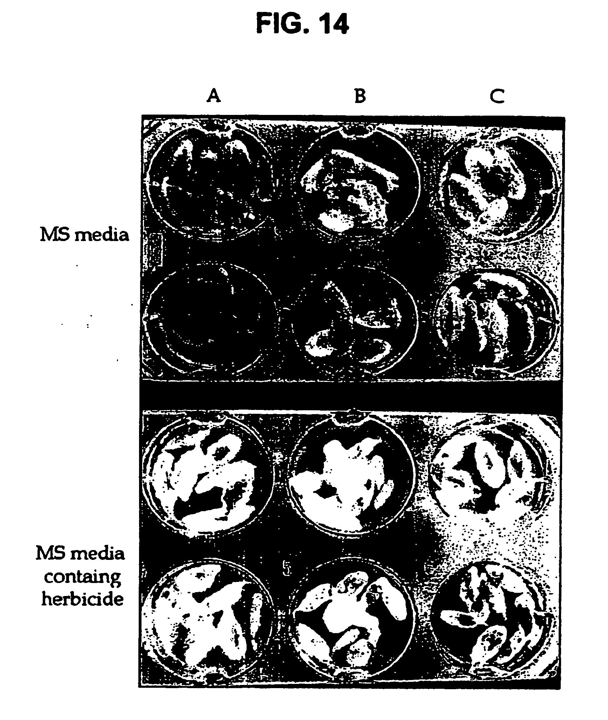

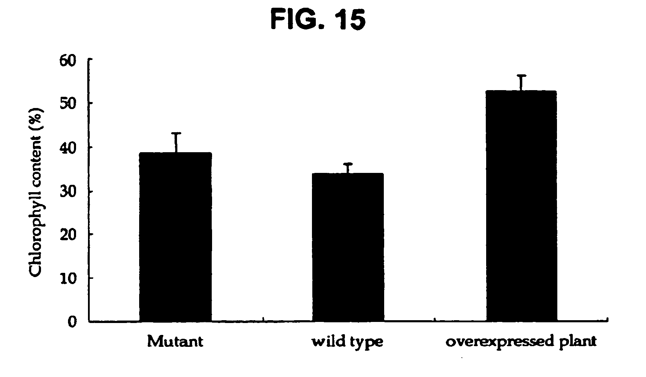

In one embodiment of the present invention, in order to examine whether the *AIA* gene is associated with the herbicide resistance of plants, the chlorophyll degradation in the *AIA* gene-overexpressed transgenic plants, caused by treatment with herbicide, was examined comparatively with wild-type plants and the mutants with inhibited expression of the *AIA* gene (see Example 7). As a result, the *AIA* gene-overexpressed transgenic plants according to the present invention showed a reduction in the chlorophyll degradation caused by herbicide, compared to wild-type *Arabidopsis thaliana* and the mutants with inhibited expression of the *AIA* gene (see FIGS. 14 and 15).

Accordingly, it could be found that the *AIA* gene has a function of enhancing the herbicide resistance of plants.

Therefore, the present invention provides a method for enhancing herbicide resistance of plants using the *AIA* gene.

Moreover, the present invention provides a method for producing herbicide-resistant plants using the *AIA* gene.

The environment stress is stress caused by herbicide. The herbicide is preferably a bipyridinium salt-based herbicide, examples of which include paraquat and diquat.

The scope of the *AIA* gene includes a polynucleotide encoding a polypeptide having an amino acid sequence shown in SEQ ID NO: 2, or a functional equivalent of the polypeptide. As used herein, the term "functional equivalent" means a polypeptide having at least 70%, preferably at least 80%, and more preferably at least 90% sequence homology to the amino acid sequence of SEQ ID NO: 2 as a result of the addition, substitution or deletion of amino acids and protein with the substantially identical physiological activity to the protein of SEQ ID NO: 2, in which the proteins have. The "substantially identical physiological activity" means an activity of controlling the environmental stress resistance of plants. For example, it means that, when the polynucleotide is overexpressed in plants, the environmental stress resistance of the plants will increase. Preferably, the polynucleotide used in the present invention has a sequence shown in SEQ IN NO: 1.

Furthermore, the present invention provides a method for producing environmental stress-resistant plants, comprising introducing a polynucleotide into the plants, wherein the polynucleotide encoding a polypeptide comprising an amino acid sequence of SEQ ID NO: 2.

The introduction of the polynucleotide into plants can be performed by inserting the polynucleotide into a suitable vector and transforming plant cells with the vector. Also, the polynucleotide sequence according to the present invention can be operably linked to an expression control sequence, and the polynucleotide sequence operably linked to the expression control sequence can be included in a single expression vector containing both a selection marker and a replication origin. The control sequence includes a promoter for performing transcription, an optional operator sequence for controlling transcription, a sequence encoding a suitable mRNA ribosome-binding site, and a sequence controlling termination. The promoter is not specifically limited as long as it can overexpress the gene inserted into plants. Examples of the promoter include, but are not limited to, the 35S RNA and 19S RNA promoters of CaMV; a figwort mosaic virus (FMV) full-length transcript promoter, and the coat protein promoter of TMV. Alternatively, an ubiquitin promoter may be used to overexpress the gene in a monocotyledon or a woody plant. Vectors suitable to introduce the inventive gene into plant cells include Ti plasmids and a plant virus vectors. Examples of the suitable vectors include, but are not limited to, binary vectors, such as pPZP, pGA and pCAMBIA series. Anyone skilled in the art can select a suitable vector for introducing the nucleic acid sequence of the inventive gene.

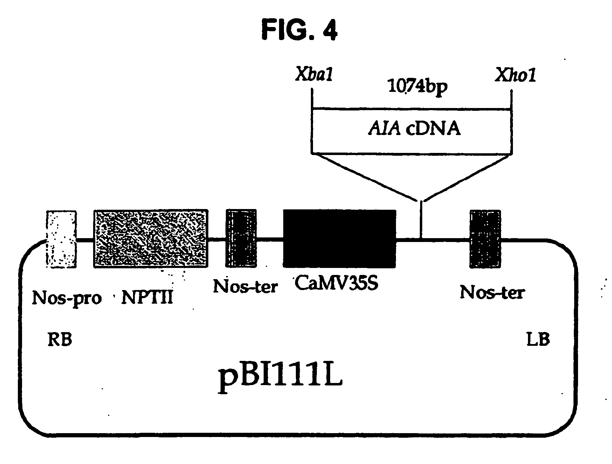

One embodiment of the present invention illustrates recombinant vector pBI111L-*AIA* prepared by inserting the *AIA* gene into vector pBI111L obtained by removing a *GUS* gene from a PBI121 vector containing a CaMV 35S promoter and an NOS terminator (see FIG. 4).

The recombinant vector according to the present invention can be introduced into plants by known methods in the art, which include but are not limited to *Agrobacterium* species-mediated transformation, particle gun bombardment, silicon carbide whiskers, sonication, electroporation and PEG (polyethyleneglycol) precipitation. In one embodiment of the present invention, *Arabidopsis thaliana* was transformed with the inventive recombinant vector using the Agrobacterium species-mediated transformation.

Also, transgenic plants transformed with the inventive polynucleotide can be selected and re-differentiated.

The selecting and re-differentiation of the transgenic plants can be performed by any method known in the art. For example, when an antibiotic-resistant gene is used as a selection marker, plants introduced with the inventive polynucleotide can be selected by incubating plants introduced with the inventive polynucleotide in an antibiotic-containing medium and selecting only plants showing antibiotic resistance. Also, whether the desired gene has been inserted into plants can additionally be confirmed using a genetic engineering technique, such as RT-PCR or Southern blot analysis.

The re-differentiation of the selected transgenic plants can be performed through the processes of callus induction, rooting and soil acclimatization according to any method known in the art.

Plants to which the inventive method can be applied include both monocotyledons and dicotyledons. Examples of the monocotyledons include, but are not limited to, rice, wheat, barley, bamboo shoot, corn, taro, asparagus, onion, garlic, Welsh onion, leek, wild rocambole, yam and ginger. Examples of the dicotyledons include, but are not limited to *Arabidopsis thaliana,* eggplant, tobacco plant, red pepper, tomato, burdock, crown daisy, lettuce, balloon flower, spinach, chard, sweet potato, celery, carrot, water dropwort, parsley, Chinese cabbage, cabbage, radish, watermelon, melon, cucumber, pumpkin, gourd, strawberry, soybean, mung beans, kidney bean, and pea. Preferably, the environmental stress-resistant plant may be *Arabidopsis thaliana.*

More specifically, the environmental stress-resistant plants according to the present invention can be produced by introducing polynucleotide into plants , wherein the polynucleotide encoding a polypeptide comprising an amino acid sequence of SEQ ID NO: 2 so as to transform the plants, selecting the transformed plants according to a conventional method and re-differentiating the selected plants. Preferably, the explants of the plants transformed with the recombinant vector comprising the *AIA* gene are placed in a suitable medium known in the art and then cultured in suitable conditions to induce the formation of calluses. When the shoots are formed, these shoots are transferred and cultured in a hormone-free medium. After about 2 weeks, the shoots are transferred to a rooting medium to induce rooting. The induced roots are transplanted and acclimated to soil, thus producing environmental stress-resistant plants.

The plant tissues or seeds are characterized by comprising a polynucleotide encoding a polypeptide comprising an amino acid sequence of SEQ ID NO: 2, and thus characterized by having resistance to environmental stress.

Influencing the expression of the inventive polynucleotide as described above preferably means promoting the expression of the polynucleotide to enhance the environmental stress resistance of plants. The effect of the test sample on the expression of the inventive polynucleotide can be measured by methods known in the art, including Northern blot analysis, Western blot analysis, etc. As used herein, the term "agent" or "test agent" includes any substance, molecule, element, compound, entity or a combination thereof. It includes, but is not limited to, e.g., proteins, polypeptides, small organic molecules, polysaccharides, polynucleotides, and the like. Also, it may be a natural product, a synthetic product, or a chemical compound, or a combination thereof. Unless otherwise specified, the terms "agent", "substance", and "compound" can be used interchangeably.

Also, either the polynucleotide encoding a polypeptide comprising an amino acid sequence of SEQ ID NO: 2 can be advantageously used with any genetic engineering technique known in the art to improve characters associated with the environmental stress resistance of plants and to investigate the environmental stress resistance-controlling gene of other plants. Preferably, the polynucleotide may be the *AIA* gene represented by SEQ ID NO: 1, and an environmental stress-resistant gene in plants can be screened using the polynucleotide as a primer or probe according to known genetic engineering techniques. Examples of the genetic engineering techniques include, but are not limited to, DNA chip, protein chip, polymerase chain reaction, Northern blot analysis, Southern blot analysis, enzyme-linked immunosorbent assay and 2-D gel analysis.

### BRIEF DESCRIPTION OF THE DRAWINGS

FIG. 1 shows the results of Northern blot analysis for the expression pattern of the *AIA* gene in each organ of wild-type *Arabidopsis thaliana.*
FIG. 2 shows the results of RT-PCR analysis for the expression pattern of the *AIA* gene in each development stage of *Arabidopsis thaliana* flowers.
   M: molecular weight marker;
   I: flower bud stage (5-6 days before pollination);
   II: pollination and flowering stage I (2-3 days before pollination);
   III: pollination and flowering stage II (pollination stage);
   IV: silique developmental stage (6-8 days after pollination); and
   V: silique maturational stage (13-16 days after pollination).
FIG. 3 shows the results of Northern blot analysis for the expression pattern of the *AIA* gene with the time passage in *Arabidopsis thaliana* treated with plant hormone ABA.
FIG. 4 shows a cleavage map of the inventive recombinant vector comprising the *AIA* gene.

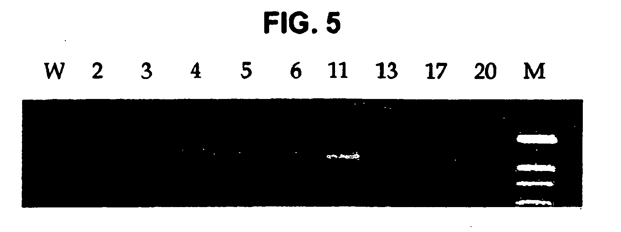

FIG. 5 shows the results of RT-PCR analysis for *Arabidopsis thaliana* transformed with the inventive recombinant vector comprising the *AIA* gene.
   W: wild-type *Arabidopsis thaliana;* and M: molecular weight marker.

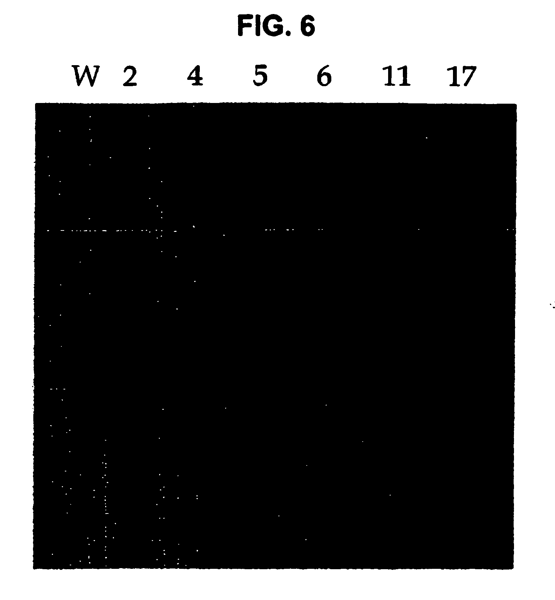

FIG. 6 shows the results of Northern blot analysis for *Arabidopsis thaliana* transformed with the inventive recombinant vector comprising the *AIA* gene.
   W: wild-type *Arabidopsis thaliana*

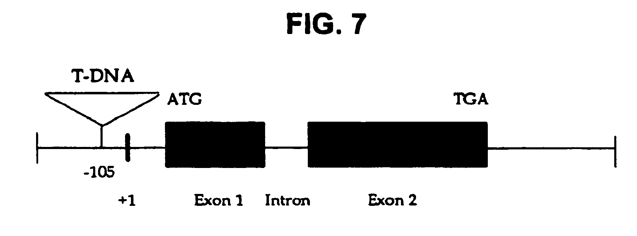

FIG. 7 shows the location of T-DNA inserted into the promoter site of the *AIA* gene.

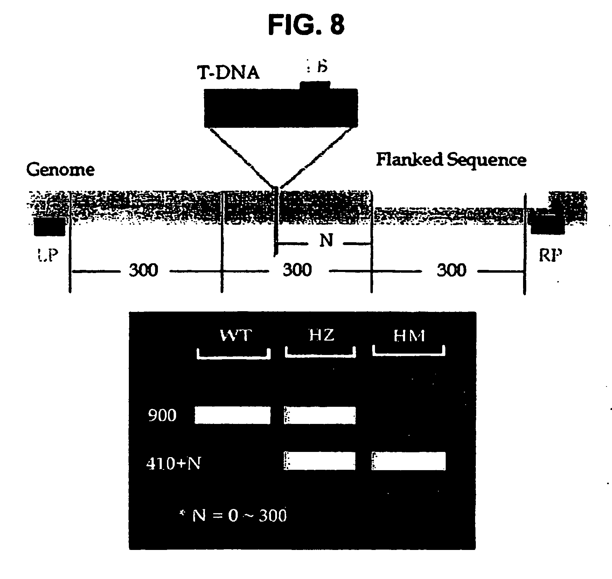

FIG. 8 shows primers designed for the selection of a homozygous mutant with inhibited activity of the *AIA* gene, and the size of a product amplified by the primers.
   WT: wild-type; HZ: heterozygous mutant; and HM: homozygous mutant.

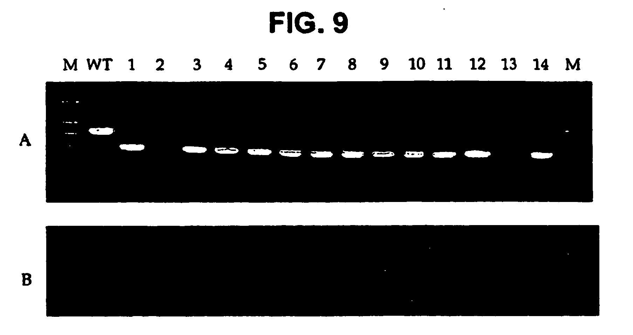

FIG. 9 shows the results of PCR conducted to select a homozygous mutant with inhibited activity of the *AIA* gene.
   A: the results of PCR amplification conducted using not only a primer placed in T-DNA but also forward and reverse primers located in the surrounding genome; and
   B: the results of PCR amplification conducted using only forward and reverse primers located in the surrounding genome without a primer located in T-DNA.

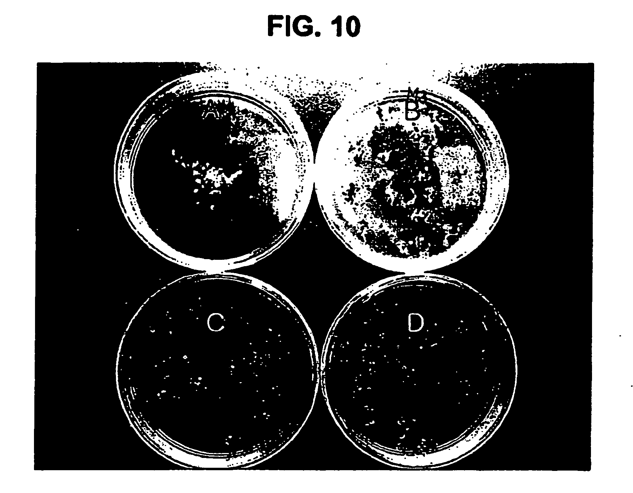

FIG. 10 shows kanamycin-resistant mutants finally selected by sowing the next-generation seeds from the homozygous mutants with inhibited activity of the *AIA* gene, which have been selected by PCR, in kanamycin-containing media.
   A: wild-type *Arabidopsis thaliana;* and B, C and D: kanamycin-resistant homozygous mutants.

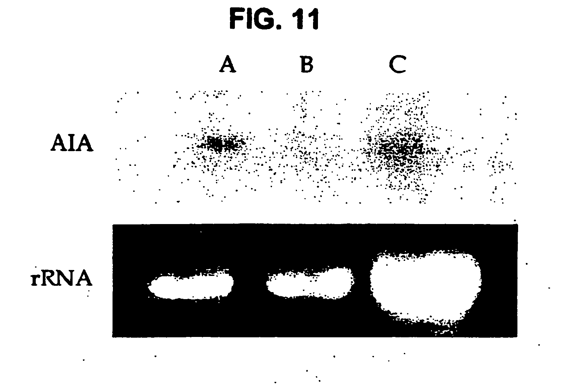

FIG. 11 shows the results of Northern blot analysis for the expression level of the *AIA* gene in homozygous mutants selected according to the inventive method.
   A: wild-type *Arabidopsis thaliana* (RNA load: 10 µg);
   B: homozygous mutant (RNA load: 10 *µ*g); and
   C: homozygous mutant (RNA load: 40 µg)
FIG. 12 illustrates photographs showing the comparison of growth states caused by drought stress between *AIA* gene-overexpressed transgenic plants, a homozygous mutant and wild-type *Arabidopsis thaliana.*
   A: wild-type *Arabidopsis thaliana;*
   B: homozygous mutant;
   C: gene-overexpressed transgenic plant 1; and
   D: gene-overexpressed transgenic plant 2.
FIG. 13 shows the measurement results for the leaf weights of an *AIA* gene-overexpressed transgenic plant, a homozygous mutant and wild-type *Arabidopsis thaliana* with the passage of time.
FIG. 14 is a photograph showing the comparison of chlorophyll degradation caused by treatment with herbicide between an *AIA* gene-overexpressed transgenic plant, a homozygous mutant and wild-type *Arabidopsis thaliana.*
FIG. 15 shows the results of quantitative analysis for the content of chlorophyll caused by treatment with herbicide in an *AIA* gene-overexpressed transgenic plant, a homozygous mutant and wild-type *Arabidopsis thaliana.*

### BEST MODE FOR CARRING OUT THE INVENTION

Hereinafter, the present invention will be described in more detail with reference to the following examples. However, the examples are given for illustrative purpose only and the scope of the present invention is not limited to or by the examples.

### Example 1: Synthesis of cDNA of AIA gene

The cDNA of the *AIA* gene was prepared by performing RT-PCR using total RNA isolated from the leaf of *Arabidopsis thaliana* as a template. The isolation of the total RNA was performed using the RNeasy plant kit (Qiagen), and the primers used in the RT-PCR reaction were designed on the basis of a known gene sequence (GenBank accession no: Atlg53910). More specifically, the RT-PCR reaction was performed using 500 ng of the above-isolated total RNA as a template, the following primers (SEQ ID NO: 3 and SEQ ID NO: 4) and a one-step RT-PCR kit (Qiagen). In the RT-PCR reaction, the reverse transcription reaction was performed at 50 °C for 30 min and at 95 °C for 15 min to yield cDNA. The PCR reaction was performed using the obtained cDNA as a template for 24 cycles of 30 sec for 94 °C, 30 sec at 60 °C and 1 min at 72 °C.
Forward primer *AIA*-ap3 (SEQ ID NO: 3)
5'-ATGTGTGGAGCTATAATATCCGAT-3'
Reverse primer *AIA*-jinw (SEQ ID NO: 4)
5'-TCAGAAGACTCCTCCAATCATGGAA T-3'

### Example 2: Analysis of expression pattern of AIA gene in wild-type Arabidopsis thaliana

The expression pattern of the *AIA* gene in each organ and development stage of wild-type *Arabidopsis thaliana* was analyzed using Northern blot and RT-PCR.

### <2-1> Northern blot analysis

Total RNA was isolated using the RNeasy plant kit (Qiagen) from the root, rosette, stem, cauline, flower and silique of *Arabidopsis thaliana* grown in soil for 4 weeks. Also, total RNA was isolated from the root and leaf of each of *Arabidopsis thaliana* grown for 4 days after germination and *Arabidopsis thaliana* grown for 2 weeks after germination.

To conduct Northern blot, 10 µg of each of the above-isolated RNAs was mixed with loading buffer (50% formamide, 1×MOPS, 2.2% formaldehyde). The mixture was loaded and separated on 1% formaldehyde agarose gel and transferred to a nylon membrane (Hybond N⁺ membrane) using 20xSSC. The nylon membrane was lightly washed with 2×SSC and then crosslinked at energy of 200 mJ. The stabilized nylon membrane was pretreated with prehybridization buffer (1% BSA, 1 mM EDTA (pH 8.0), 0.25 M Na₂HPO₄ (pH 7.2), 7% SDS) for 3 hours and then hybridized with a labeled probe specific to the *AIA* gene at 65 °C overnight. The probe was prepared by PCR-amplifying the 3'UTR region of the *AIA* gene using the *AIA* gene cDNA (SEQ ID NO: 1) prepared in Example 1 as a template and the following primers (SEQ ID NO: 5 and SEQ ID NO: 6) and it was labeled with [α-³²P]dCTP.
Forward primer (SEQ ID NO: 5)
5'-CGTTGATGCTGGATGTAATGGGTAT-3'
Reverse primer (SEQ ID NO: 6)
5'-CCTGAGTCGTTACAGCATCTTCGTT-3'

After completion of the hybridization, the nylon membrane was washed with 2×SSC and 0.1% SDS at 65 °C for 5 minutes and then exposed to an X-ray film to detect bands sensitized with radiations.

The test results showed that the *AIA* gene was expressed in all the organs of wild-type *Arabidopsis thaliana,* and particularly, highly expressed in roots and flowers compared to other organs (see FIG. 1).

Also, it was shown that the *AIA* gene was expressed in both the leaves and roots of *Arabidopsis thaliana* grown for 4 days after germination and 2 weeks after germination (data not shown).

From the above test results, it was inferred that the *AIA* gene would perform a specific function throughout the life cycle of plants while existing in all the organs of the plants.

### <2-2> RT-PCR analysis

On the basis of the result of Example 2-1 indicating that the *AIA* gene is highly expressed in the flower of *Arabidopsis thaliana,* the expression pattern of the *AIA* gene in each developmental stage of *Arabidopsis thaliana* flowers was analyzed by RT-PCR. For this purpose, from the flower of *Arabidopsis thaliana* grown in soil for 4 weeks, total RNA was isolated in the same manner as in Example 1, in each of the flower developmental stages (5-6 days before pollination, 2-3 days before pollination, the pollination stage, 6-8 days after pollination and 13-16 days after pollination). The isolated total RNA was subjected to RT-PCR in the same manner as in Example 1.

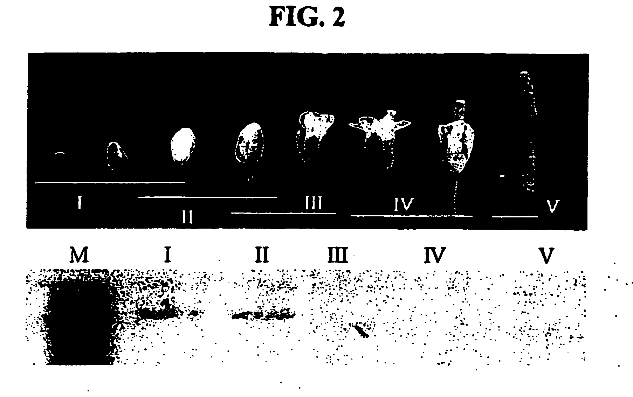

The test results showed that the *AIA* gene was most highly expressed before pollination, and the expression level was gradually decreased with the progression of the development stages (see FIG. 2).

### Example 3: Changes in expression of AIA gene in Arabidopsis thaliana, caused by treatment with plant hormones

*Arabidopsis thaliana* was grown in MS medium (2.15 g MS salt, 0.5 g MES, 10 g sucrose, 0.7% phytoagar, pH 5.7) for 2 weeks and then sprayed with an aqueous solution containing 100 (M of each of plant hormones, ABA, jasmonic acid and ethephon and 10 (M of each of auxin and gibberellic acid (GA). Next, total RNA was isolated from the seedling of *Arabidopsis thaliana* at varying time points (30 min, 2 hr, 6 hr, 10 hr and 24 hr after the spraying) in the same manner as in Example 2-1 and subjected to Northern blot analysis. In the analysis, a *KIN2* gene known to have an increase in the expression level thereof due to ABA was used as a positive control group.

The test results showed that, in the case of treatment with ABA, the AL4 gene in *Arabidopsis thaliana* was expressed at a higher level than the initial level from 2 hours after the spraying, and the expression level thereof was reduced to approximately the initial expression level at 24 hours after the spraying (see FIG. 3). On the other hand, in the cases of treatment with hormones, such as auxin, gibberellic acid, jasmonic acid and ethephon, the expression level of the *AIA* gene was not greatly different from the initial level (data not shown).

It is generally known that drought resistance-associated genes are induced by treatment with ABA. Thus, it was inferred from the above test results that the *AIA* gene would also be associated with the drought resistance of plants.

### Example 4: Production of transgenic plants overexpressing AIA gene

In order to confirm whether the *AIA* gene is associated with the environmental stress resistance of plants, transgenic plants overexpressing the *AIA* gene were produced.

### <4-1> Preparation of recombinant vector containing AIA gene

To produce transgenic plants overexpressing the *AIA* gene, the *AIA* gene cDNA prepared in Example 1 was digested with restriction enzymes *XbaI* and *XhoI* and inserted into the same sites of a pBI111L vector comprising a CaMV 35S promoter and an NOS terminator, thus preparing recombinant vector pBI111L*-AIA* (see FIG. 4).

### <4-2> Transformation of Arabidopsis thaliana

The *AIA* gene-comprising recombinant vector prepared in Example 4-1 was introduced into *Agrobacterium tumefaciens* C58C1 by an electroporation method using a micropulser electroporator (Biorad). Then, the *Agrobacterium tumefaciens* was cultured in YEP medium (containing 10 g/L yeast extract, 10 g/L peptone and 5 g/L NaCl) supplemented with kanamycin, gentamycine and rifambicin at final concentrations of 30 µg/ml, 100 µg/ml and 100 µg/ml, respectively, so as to select a transgenic strain showing antibiotic resistance. *Arabidopsis thaliana* ecotype Col-0 was transformed with the selected *Agrobacterium* strain by the floral dip method (Clough et al., Plant J., 16(6):735-743, 1998). Namely, the *Agrobacterium* strain introduced with the *AIA* gene-comprising recombinant vector was inoculated and cultured in 5 ml of the YEP medium containing kanamycin, gentamycine and rifambicin for one day. 500 µℓ of the culture medium was taken, and inoculated in 500 ml of YEP medium having the same conditions, and then cultured to an absorbance of more than 2.0 at 600 nm. After completion of the culture, the culture medium was centrifuged, and the precipitated cells were collected and suspended in infiltration buffer (2.2 g/L MS salt, 50 g/L sucrose, 0.5 g/L MES, 0.044 M benzylaminopurin, and 200 µℓ/L Silwet L-77, pH 5.7). *Arabidopsis thaliana* grown for 4 weeks was immersed three times in the suspension for 5-7 seconds each time and covered with a wrap, followed by culture for 24 hours. The *Arabidopsis thaliana* transformed as described above was continued to culture, so as to harvest the next-generation seeds. The harvested seeds were sown in a kanamycin-containing MS medium and cultured for 7 days, thus selecting 9 plants showing kanamycin resistance.

### <4-3> RT-PCR of transgenic Arabidopsis thaliana

*9 Arabidopsis thaliana* plants selected in Example <4-2> were transferred and grown in soil for 4 weeks, and the rosette leaves were collected. Total RNA was isolated from the rosette leaves in the same manner as in Example 1 and subjected to RT-PCR so as to examine whether the *AIA* gene was overexpressed.

In the test results, the *AIA* gene band was shown in six plants of the nine transgenic plants, in which the band intensity of No. 11 transgenic plant among them was the strongest (see FIG. 5).

### <4-4> Southern blot analysis of transgenic Arabidopsis thaliana

Genomic DNA was extracted from the six transgenic plants having the *AIA* gene band detected in Example <4-3> and then subjected to Southern blot to analyze whether the *AIA* gene was inserted into the genomes of the transgenic plant. To extract the genomic DNA, about 2 g of the leaves and stem of a 6-week-old T1 transgenic plant were detached, crushed with liquid nitrogen and then left to stand in 10 ml of CTAB buffer [2% (w/v) CTAB (cetyltrimethylammonium bromide), 100 mM Tris-HCl, pH 8.0, 20 mM EDTA, pH8.0, 1.4 M NaCl and 1% polyvinylpyrrolidine (M.W. 40,000)] at 65 °C for about 3 hours. After completion of the reaction, the same amount of chloroform was carefully added to the reaction substance, followed by centrifugation at 3000 rpm for 5 minutes. The supernatant was transferred to a new tube and mixed with a 2/3 volume of isopropanol, followed by centrifugation at 12000 rpm for 5 minutes. After removing the supernatant, the precipitate was washed with 70% ethanol, and genomic DNA was first extracted from the precipitate using TE buffer. To remove the remaining RNA, RNase A (promega, USA) was added to the extracted substance to a final concentration of 10 µg/ml and the mixture was cultured at 37 °C for 30 minutes, and then, pure genomic DNA was extracted using an ethanol extraction method. For Southern blot analysis, 10 µg of the genomic DNA collected from each of the transgenic plants was taken, and digested with restriction enzymes *EcoRI* and *PstI* for 24 hours and then loaded on 0.7% agarose gel. After completion of the loading, the agarose gel was precipitated in 300 ml of modification buffer (1.5 M NaCl, 0.5 N NaOH) and lightly stirred for 30 minutes. Then, the buffer was discarded and the agarose gel was washed with double-distilled water. Then, the agarose gel was precipitated in neutralization buffer (1 M Tris-HCl (pH7.5), 1.5 M NaCl) for 30 minutes and subjected to Southern blot analysis in the same manner as described for the above Northern blot analysis. The crosslinked nylon membrane was pretreated with a prehybridization buffer (1% SDS, 1 M NaCl, 10% dextran sulfate) for Southern blot for 3 hours, and then hybridized with a probe specific to a 35S CaMV promoter at 65 °C overnight. The probe was prepared by PCR amplification using the 35S CaMV promoter present in vector pBI111L as a template and the following primers (SEQ ID NO: 7 and SEQ ID NO: 8), and was labeled with [α-³²P]dCTP.
Forward primer (SEQ ID NO: 7)
5'-CTAACTGCATCAAGAACACAGAGAA-3'
Reverse primer (SEQ ID NO: 8)
5'-AGATATCACATCAATCCACTTGCTT-3'

After completion of the hybridization, the nylon membrane was washed with 2xSSC and 0.1% SDS at 65 °C for 5 minutes and exposed to an X-ray film to detect bands sensitized with radiations.

In the test results, Nos. 2, 4 and 11 transgenic plants showed one strong band although they sometimes showed weak bands (see FIG. 6). Accordingly, it could be found that the *AIA* gene is present as a single-copy gene in the genome of *Arabidopsis thaliana.*

### Example 5: Production of mutants with inhibited activity of AIA gene

### <5-1> Production of homozygous mutant

In order to examine whether the *AIA* gene is associated with the environmental stress resistance of plants, a homozygous mutant with inhibited activity of the *AIA* gene was produced. For this purpose, seeds (Salk_019187) having T-DNA inserted into the promoter region of the *AIA* gene were obtained from Salk (http://signal.salk.edubin/tdnaexpress) (see FIG. 7). The seeds were sown in a kanamycin-containing MS medium, and after 2 weeks, transplanted into soil and additionally grown for 2 weeks. Then, genomic DNA was isolated from the leaves of the grown plants. The isolation of the genomic DNA was performed in the following manner. Two leaves of *Arabidopsis thaliana* were placed in an E-tube into which liquid nitrogen was then charged. The leaves were chopped using a plastic probe, to which 500 µℓ of extraction buffer [100 mM Tris-HCl (pH8.5), 50 mM EDTA (pH8.5) and 500 mM NaCl] and 20% 35 µℓ of SDS were then added. The mixture was left to stand at 65 °C for 5 minutes. To the mixture, 130 µℓ of 5 M KOAc was added and then left to stand on ice for 5 minutes, followed by centrifugation at 15000 rpm for 10 minutes. The collected supernatant was transferred into a new tube to which 640 µℓ of isopropanol and 60 µℓ of 3 M NaOAc were then added. The mixture was cultured at -20 °C for 10 minutes and centrifuged at 15000 rpm for 10 minutes. The DNA precipitate was washed with 70% ethanol and genomic DNA was extracted from the washed precipitate using double-distilled water. The genomic DNA template obtained as described above was amplified by PCR using the following primers (SEQ ID NO: 9, SEQ ID NO: 10 and SEQ ID NO: 11). The PCR amplification product was electrophoresed so as to select homozygous mutants having T-DNA inserted therein. The primers used in the PCR amplification reaction were primer LB located in T-DNA, and forward primer LP and reverse primer RP located in the surrounding genome. Also, the PCR amplification reaction was performed for 35 cycles of 30 sec at 94 °C, 30 sec at 56 °C and 2 min at 72 °C. In addition, another PCR amplification was performed in the same conditions using only the forward and reverse primers without the primer located in T-DNA, and the PCR amplification product was electrophoresed.
LB primer (SEQ ID NO: 9)
5'- GCGTGGACCGCTTGCTGCAACT-3'
LP primer (SEQ ID NO: 10)
5'-TGTTAAATATCTGTTCTGCGTTGGC-3'
RP primer (SEQ ID NO: 11)
5'-AACCAATTCAATCGCTCAAAC-3'

When the PCR products amplified using all the three kinds of primers as described above were electrophoresed, one band having a 900-bp size (from LP to RP) was detected in the wild type plant. Also, in the case of the homozygous mutant, one band having a size of 410+N bp (N=0-300)(300+N bases from RP and 110 bases from LB to the left border of the vector) was detected. However, the whole-genome amplification product having T-DNA inserted therein was not detected in electrophoresis because it was too large in size. In the case of heterozygous mutant, both a 900-bp size band and a 410+N bp size band were detected, because one strand of the chromosome was identical to the wild-type plant, and T-DNA was inserted into only the remaining one strand (see FIG. 8).

Meanwhile, when the PCR products amplified using the forward and reverse primers located in the surrounding genome without the primer located in T-DNA were electrophoresed, a 900-bp size band was detected in the wild-type band, whereas the mutant having the genome containing T-DNA inserted therein was not detected in electrophoresis because the amplification product was too large in size.

As a result of PCR amplification and electrophoresis conducted using all the three kinds of primers according to the method and principle as described above, 12 homozygous mutants were selected from a total of 14 plants (see FIG. 9). Meanwhile, the results of electrophoresis for the PCR products amplified using only the primers located in the surrounding genome without the primer located in T-DNA showed that a 900-bp band was detected only in the wild-type plant (see FIG. 9).

The next-generation seeds from the 12 mutants selected as described above were harvested and then sown in kanamycin-containing MS media so as to finally select a kanamycin-resistant mutant. The selected kanamycin-resistant mutant was named *"aia* " (see FIG. 10).

### <5-2> Measurement of expression level of AIA gene in homozygous mutant

The mutant (*aia*) selected in Example 5-1 was grown in soil for 4 weeks, after which total RNA was isolated from the leaves and subjected to Northern blot analysis in the same manner as in Example 2-1 so as to measure the expression level of the *AIA* gene.

In the test results, it was shown that the expression level of the *AIA* gene in the homozygous mutant (*aia*) was much lower than in the wild-type plant. Also, when the RNA of the homozygous mutant was loaded at about 4-fold higher concentration than that of the wild-type plant, it showed an expression level similar to the wild-type plant (see FIG. 11). Accordingly, it could be found that the expression of the *AIA* gene in the homozygous mutant was inhibited.

### Example 6: Examination of drought resistance of transgenic plants with overexpressed AIA gene

In order to examine whether the *AIA* gene is associated with the drought resistance of plants, the growth state and transpiration rate of the *AIA* gene-overexpressed transgenic plant, caused by drought stress, were examined comparatively with a wild-type plant and a mutant with inhibited expression of the *AIA* gene.

### <6-1> Comparison of growth state caused by drought stress

Culturing of wild-type *Arabidopsis thaliana,* the *AIA* gene-overexpressed transgenic plant produced in Example <4-2>, and the mutant produced in Example <5-1>, was continued so as to harvest the next-generation seeds. 100 seeds for each of the plants were sown in soil and grown in long-day conditions (16-hr light/8-hr dark) at 23 °C for 2 weeks. Then, water supply was stopped for 10 days to apply drought stress to the plants, after which the plants were supplied again with water, and after two days, observed for growth state.

The test results showed that the wild-type *Arabidopsis thaliana* plants and homozygous mutants showed a death rate of more than 80%. On the other hand, the *AIA* gene-overexpressed transgenic mutants mostly survived (see FIG. 12).

### <6-2> Comparison of transpiration rate between plants

In order to more specifically examine the drought resistance of the *AIA* gene-overexpressed transgenic plant according to the present invention, the following test was performed. Wild-type *Arabidopsis thaliana,* the *AIA* gene-overexpressed transgenic plant produced in Example 4-2 and the mutant produced in Example 5-1 were grown and Nos. 3, 4 and 5 leaves of the plants were detached and placed on 3M papers such that the abaxial sides of the leaves faced upward. The weight of the leaves was measured at one-hour intervals for 6 hours so as to compare transpiration rates with the passage of time.

In the test results, the *AIA* gene-overexpressed transgenic plant was smaller in water loss than the wild-type plant and the mutant. Namely, after 6 hours, the leaf weight of the transgenic plant was maintained at about 70% of the initial weight, whereas the leaf weight of each of the wild-type plant and the mutant was reduced to a approximately less than 50% (see FIG. 13).

Accordingly, it could be found that the transpiration of the *AIA* gene-overexpressed transgenic plant was inhibited compared to the wild-type plant or the mutant with inhibited expression of the *AIA* gene, so as to show an increased resistance to drought stress.

### Example 7: Examination of herbicide resistance of AIA gene-overexpressed transgenic plant

In order to examine whether the *AIA* gene is associated with the herbicide resistance of plants, the chlorophyll degradation in the *AIA* gene-overexpressed transgenic plant, caused by treatment with herbicide, was examined comparatively with those in the wild-type plant and the mutant with inhibited expression of the *AIA* gene.

The leaves of wild-type *Arabidopsis thaliana,* the *AIA* gene-overexpressed transgenic plant produced in Example 4-2 and the mutant produced in Example 5-1, which had been grown for 4 weeks, were floated on MS media containing 6 µM of herbicide paraquat (methyl viologen) and cultured in continuous light conditions at 23 °C for 72 hours. For a control group, an MS medium containing no methyl viologen was used. The methyl viologen, which is a nonselective herbicide commercially available under the trademark "Gramoxone", is a toxic substance that can not only wither all plants having chlorophyll, but also cause fatal problems when absorbed into the human body.

The chlorophyll degradation caused by herbicide stress was measured in the following manner. First, 2 g of the leaves of each of the plants cultured as described above were collected and crushed in a mortar and pestle while adding 85% acetone in portions together with 0.1 g CaCO₃. Then, the solution was centrifuged at 3000 rpm for 5 minutes. After separating the supernatant, chlorophyll was quantified according to a known method (Cunniff.P.A. Plant in Official Method of Analysis. vol1. 16th ed., pp26-28, 1995).

The test results showed that, when the leaves were cultured in the herbicide-containing medium, the wild-type *Arabidopsis thaliana* and the homozygous mutant had an increase of more than 60% in chlorophyll degradation compared to the control group, whereas the *AIA* gene-overexpressed transgenic plant had a reduction of about 10-20% in chlorophyll degradation compared to the wild-type plant and the mutant (see FIGS. 14 and 15).

## Claims

1. A method for enhancing the environmental stress resistance of plants, consisting of introducing an AIA (ABA Inducible AP2/ERF transcription factor) polynucleotide-comprising recombinant vector into the plants, wherein the polynucleotide encodes a polypeptide comprising an amino acid sequence of SEQ ID NO: 2, and wherein the environmental stress is paraquat or diquat.

2. The method of Claim 1, wherein the polynucleotide has the sequence of SEQ ID NO: 1.

3. The method of Claim 1, wherein the introduction of the recombinant vector into the plants is performed using any one selected from the group consisting of *Agrobacterium sp.-*mediated transformation, particle gun bombardment, silicon carbide whiskers, sonication, electroporation and PEG (polyethylene glycol) precipitation.

4. The method of Claim 1, wherein the plants are monocotyledons or dicotyledons.

5. The method of Claim 4, wherein the monocotyledons are selected from the group consisting of rice, wheat, barley, bamboo shoot, corn, taro, asparagus, onion, garlic, Welsh onion, leek, wild rocambole, yam and ginger.

6. The method of Claim 4, wherein the dicotyledons are selected from the group consisting of *Arabidopsis thaliana,* eggplant, tobacco plant, red pepper, tomato, burdock, crown daisy, lettuce, balloon flower, spinach, chard, sweet potato, celery, carrot, water dropwort, parsley, Chinese cabbage, cabbage, radish, watermelon, melon, cucumber, pumpkin, gourd, strawberry, soybean, mung beans, kidney bean, and pea.

7. A method for producing environmental stress-resistant plants, consisting of introducing an AIA (ABA Inducible AP2/ERF transcription factor) polynucleotide-comprising recombinant vector into the plants, wherein the polynucleotide encodes a polypeptide comprising an amino acid sequence of SEQ ID NO: 2, wherein the environmental stress is a herbicide selected from the group consisting of paraquat or diquat.

8. The method of Claim 7, wherein the polynucleotide has a sequence of SEQ ID NO: 1.

## Patentansprüche

1. Verfahren zur Verbesserung der umweltbedingten Stressresistenz von Pflanzen, welches Einführen eines AIA (ABA induzierbarer AP2/ERF Transkriptionsfaktor) Polynucleotidumfassenden rekombinanten Vektors in die Pflanzen umfasst, wobei das Polynucleotid für ein Polypeptid kodiert, welches eine Aminosäuresequenz der SEQ ID NO: 2 umfasst, und wobei der umweltbedingte Stress paraquat oder diquat ist.

2. Verfahren nach Anspruch 1, wobei das Polynucleotid die Sequenz der SEQ ID NO: 1 aufweist.

3. Verfahren nach Anspruch 1, wobei das Einführen des rekombinanten Vektors in die Pflanzen unter Verwendung irgendeines, ausgesucht aus der Gruppe bestehend aus *Agrobacterium sp*.-vermittelter Transformation, Partikelkanonenbeschuss, Silikonkarbid-Fasern, Beschallung, Elektroporation und PEG (Polyethylenglycol) Ausfällung durchgeführt wird.

4. Verfahren nach Anspruch 1, wobei die Pflanzen einkeimblättrig oder zweikeimblättrig sind.

5. Verfahren nach Anspruch 4, wobei die Einkeimblättrigen aus der Gruppe bestehend aus Reis, Weizen, Gerste, Bambussprossen, Getreide, Taro, Spargel, Zwiebel, Knoblauch, walisischer Zwiebel, Lauch, wilder Rocambole, Jamswurzel und Ingwer ausgewählt sind.

6. Verfahren nach Anspruch 4, wobei die Zweikeimblättrigen aus der Gruppe bestehend aus *Arabidopsis thaliana,* Aubergine, Tabakpflanze, roter Pfeffer, Tomate, große Klette, Kronen-Wucherblume, Salat, Ballonblume, Spinat, Mangold, Süßkartoffel, Sellerie, Karotte, Wasserfenchel, Petersilie, Chinakohl, Kohl, Rettich, Wassermelone, Melone, Gurke, Kürbis, Flaschenkürbis, Erdbeere, Sojabohne, Mungbohne, Kidneybohne und Erbse ausgewählt sind.

7. Verfahren zur Herstellung von umweltbedingt stressresistenten Pflanzen, welches Einführen eines AIA (ABA induzierbarer AP2/ERF Transkriptionsfaktor) Polynucleotidumfassenden rekombinanten Vektors in die Pflanzen umfasst, wobei das Polynucleotid für ein Polypeptid kodiert, welches eine Aminosäuresequenz der SEQ ID NO: 2 aufweist, wobei der umweltbedingte Stress ein Herbizid ausgewählt aus der Gruppe bestehend aus paraquat oder diquat ist.

8. Verfahren nach Anspruch 7, wobei das Polynucleotid eine Sequenz der SEQ ID NO: 1 aufweist.

## Revendications

1. Procédé permettant d'améliorer la résistance des plantes aux stress environnementaux, consistant à introduire dans les plantes un vecteur recombinant comprenant un polynucléotide d'AIA (facteur de transcription AP2/ERF inductible d'ABA), dans lequel le polynucléotide code un polypeptide comprenant une séquence d'acides aminés de SEQ ID NO : 2, et dans lequel le stress environnemental est le paraquat ou le diquat.

2. Procédé selon la revendication 1, dans lequel le polynucléotide présente la séquence de SEQ ID NO: 1.

3. Procédé selon la revendication 1, dans lequel l'introduction du vecteur recombinant dans les plantes est réalisée en utilisant l'une quelconque des procédures choisie dans le groupe constitué par une transformation par l'intermédiaire d'Agrobacterium sp., un bombardement par canon à particules, des wiskers de carbure de silicium, une sonification, une électroporation et une précipitation de PEG (polyéthylène - glycol).

4. Procédé selon la revendication 1, dans lequel les plantes sont des monocotylédones ou des dicotylédones.

5. Procédé selon la revendication 4, dans lequel les monocotylédones sont choisies dans le groupe constitué de riz, blé, orge, pousse de bambou, maïs, taro, asperge, oignon, ail, oignon du Pays de Galles, poireau, échalote sauvage, igname et gingembre.

6. Procédé selon la revendication 4, dans lequel les dicotylédones sont choisies dans le groupe constitué de *Arabidopsis thaliana,* aubergine, plant de tabac, poivre rouge, tomate, bardane, reine-marguerite, laitue, chou-fleur, épinard, bette, pomme de terre douce, céleri, carotte, reine des prés, persil, chou chinois, chou, radis, melon Santa Claus, melon, concombre, citrouille, calebasse, fraise, soja, germes de haricot, flageolet et pois.

7. Procédé de production de plantes résistantes au stress environnemental, consistant à introduire dans les plantes un vecteur recombinant comprenant un polynucléotide d'AIA (facteur de transcription AP2/ERF inductible d'ABA), dans lequel le polynucléotide code un polypeptide comportant une séquence d'acides aminés de SEQ ID NO: 2, dans lequel le stress environnemental est un herbicide choisi dans le groupe constitué de paraquat ou de diquat.

8. Procédé selon la revendication 7, dans lequel le polynucléotide présente une séquence de SEQ ID NO: 1.


### SEQUENCE LISTING

<110> Seoul National University Industry Foundation
<120> METHOD FOR ENHANCING ENVIRONMENTAL STRESS RESISTANCE OF PLANT USING ENVIRONMENTAL STRESS CONTROLLING GENE
<150> KR10-2005-0007534
   <151> 2005-01-27
<160> 11
<170> Kopatent In 1.71
<210> 1
   <211> 1077
   <212> DNA
   <213> Arabidopsis thaliana
<220>
   <221> COS
   <222> (1)..(1074)
   <223> cDNA of *AIA* gene
<400> 1
   atg tgt gga gga gct ata ata tcc gat ttc att cca ccg ccg agg tct    48
   Met Cys Gly Gly Ala Ile Ile Ser Asp Phe Ile Pro Pro Pro Arg Ser
   1               5                   10                  15
   cgc cgt gtt act agc gag ttt att tgg ccg gat ctg aag aag aat ttg    96
   Arg Arg Val Thr Ser Glu Phe Ile Trp Pro Asp Leu Lys Lys Asn Leu
               20                  25                  30
   aaa gga tcg aag aaa agc tcg aag aat cgt tcg aat ttc ttc gat ttt    144
   Lys Gly Ser Lys Lys Ser Ser Lys Asn Arg Ser Asn Phe Phe Asp Phe
           35                  40                  45
   gac gct gag ttc gaa gct gat ttc caa ggt ttc aaa gat gat tcg tct    192
   Asp Ala Glu Phe Glu Ala Asp Phe Gln Gly Phe Lys Asp Asp Ser Ser
       50                  55                  60
   atc gat tgc gat gat gat ttc gac gtc ggt gat gtt ttc gcc gat gtg    240
   Ile Asp Cys Asp Asp Asp Phe Asp Val Gly Asp Val Phe Ala Asp Val
   65                  70                  75                  80
   aaa cca ttc gtt ttc act tcg act cca aaa ccc gcc gtc tcc gcc gct    288
   Lys Pro Phe Val Phe Thr Ser Thr Pro Lys Pro Ala Val Ser Ala Ala
                   85                  90                  95
   gcg gaa ggt tca gtt ttt ggt aag aaa gtt act ggc ttg gat ggg gac      336
   Ala Glu Gly Ser Val Phe Gly Lys Lys Val Thr Gly Leu Asp Gly Asp
               100                 105                 110
   gct gag aaa tct gca aat agg aag agg aag aat cag tac cga ggg att      384
   Ala Glu Lys Ser Ala Asn Arg Lys Arg Lys Asn Gln Tyr Arg Gly Ile
           115                 120                 125
   agg caa cgt cct tgg gga aaa tgg gct gct gag ata cgt gat cca agg      432
   Arg Gln Arg Pro Trp Gly Lys Trp Ala Ala Glu Ile Arg Asp Pro Arg
       130                 135                 140
   gaa ggt gct aga atc tgg ctt gga acg ttc aag aca gct gag gaa gct      480
   Glu Gly Ala Arg Ile Trp Leu Gly Thr Phe Lys Thr Ala Glu Glu Ala
   145                 150                 155                 160
   gct aga gct tac gat gct gca gcg cgg aga atc cgt gga tct aaa gct      528
   Ala Arg Ala Tyr Asp Ala Ala Ala Arg Arg Ile Arg Gly Ser Lys Ala
                   165                 170                 175
   aag gtg aat ttc cct gaa gaa aac atg aag gct aat tct cag aaa cgc      576
   Lys Val Asn Phe Pro Glu Glu Asn Met Lys Ala Asn Ser Gln Lys Arg
               180                 185                 190
   tct gtg aag gct aat ctt cag aaa cca gtg gct aaa cct aac cct aac      624
   Ser Val Lys Ala Asn Leu Gln Lys Pro Val Ala Lys Pro Asn Pro Asn
           195                 200                 205
   cca agt cca gct ttg gtt cag aac tcg aac atc tcc ttt gaa aat atg      672
   Pro Ser Pro Ala Leu Val Gln Asn Ser Asn Ile Ser Phe Glu Asn Met
       210                 215                 220
   tgt ttc atg gag gag aaa cac caa gtg agc aac aac aac aac aac cag      720
   Cys Phe Met Glu Glu Lys His Gln Val Ser Asn Asn Asn Asn Asn Gln
   225                 230                 235                 240
   ttt ggg atg aca aac tcc gtt gat gct gga tgt aat ggg tat cag tat      768
   Phe Gly Met Thr Asn Ser Val Asp Ala Gly Cys Asn Gly Tyr Gln Tyr
                   245                 250                 255
   ttc agc tct gac cag ggt agt aat tct ttc gat tgt tcg gag ttt ggt      816
   Phe Ser Ser Asp Gln Gly Ser Asn Ser Phe Asp Cys Ser Glu Phe Gly
               260                 265                 270
   tgg agc gat caa gct ccg ata act ccc gac atc tct tct gcg gtt atc      864
   Trp Ser Asp Gln Ala Pro Ile Thr Pro Asp Ile Ser Ser Ala Val Ile
           275                 280                 285
   aac aac aac aac tca gct ctg ttc ttt gag gaa gcc aat cca gct aag      912
   Asn Asn Asn Asn Ser Ala Leu Phe Phe Glu Glu Ala Asn Pro Ala Lys
   290 295 300
   aag ctc aag tct atg gat ttc gag aca cct tac aac aac act gaa tgg 960
   Lys Leu Lys Ser Met Asp Phe Glu Thr Pro Tyr Asn Asn Thr Glu Trp
   305 310 315 320
   gac gct tca ctg gat ttc ctc aac gaa gat gct gta acg act cag gac 1008
   Asp Ala Ser Leu Asp Phe Leu Asn Glu Asp Ala Val Thr Thr Gln Asp
   325 330 335
   aat ggt gca aac cct atg gac cta tgg agt att gat gaa att cat tcc 1056
   Asn Gly Ala Asn Pro Met Asp Leu Trp Ser Ile Asp Glu Ile His Ser
   340 345 350
   atg att gga gga gtc ttc tga 1077
   Met Ile Gly Gly Val Phe
   355
<210> 2
   <211> 358
   <212> PRT
   <213> Arabidopsis thaliana
<400> 2
   Met Cys Gly Gly Ala Ile Ile Ser Asp Phe Ile Pro Pro Pro Arg Ser
   1 5 10 15
   Arg Arg Val Thr Ser Glu Phe Ile Trp Pro Asp Leu Lys Lys Asn Leu
   20 25 30
   Lys Gly Ser Lys Lys Ser Ser Lys Asn Arg Ser Asn Phe Phe Asp Phe
   35 40 45
   Asp Ala Glu Phe Glu Ala Asp Phe Gln Gly Phe Lys Asp Asp Ser Ser
   50 55 60
   Ile Asp Cys Asp Asp Asp Phe Asp Val Gly Asp Val Phe Ala Asp Val
   65 70 75 80
   Lys Pro Phe Val Phe Thr Ser Thr Pro Lys Pro Ala Val Ser Ala Ala
   85 90 95
   Ala Glu Gly Ser Val Phe Gly Lys Lys Val Thr Gly Leu Asp Gly Asp
   100 105 110
   Ala Glu Lys Ser Ala Asn Arg Lys Arg Lys Asn Gln Tyr Arg Gly Ile
   115 120 125
   Arg Gln Arg Pro Trp Gly Lys Trp Ala Ala Glu Ile Arg Asp Pro Arg
   130 135 140
   Glu Gly Ala Arg Ile Trp Leu Gly Thr Phe Lys Thr Ala Glu Glu Ala
   145 150 155 160
   Ala Arg Ala Tyr Asp Ala Ala Ala Arg Arg Ile Arg Gly Ser Lys Ala
   165 170 175
   Lys Val Asn Phe Pro Glu Glu Asn Met Lys Ala Asn Ser Gln Lys Arg
   180 185 190
   Ser Val Lys Ala Asn Leu Gln Lys Pro Val Ala Lys Pro Asn Pro Asn
   195 200 205
   Pro Ser Pro Ala Leu Val Gln Asn Ser Asn Ile Ser Phe Glu Asn Met
   210 215 220
   Cys Phe Met Glu Glu Lys His Gln Val Ser Asn Asn Asn Asn Asn Gln
   225 230 235 240
   Phe Gly Met Thr Asn Ser Val Asp Ala Gly Cys Asn Gly Tyr Gln Tyr
   245 250 255
   Phe Ser Ser Asp Gln Gly Ser Asn Ser Phe Asp Cys Ser Glu Phe Gly
   260 265 270
   Trp Ser Asp Gln Ala Pro Ile Thr Pro Asp Ile Ser Ser Ala Val Ile
   275 280 285
   Asn Asn Asn Asn Ser Ala Leu Phe Phe Glu Glu Ala Asn Pro Ala Lys
   290 295 300
   Lys Leu Lys Ser Met Asp Phe Glu Thr Pro Tyr Asn Asn Thr Glu Trp
   305 310 315 320
   Asp Ala Ser Leu Asp Phe Leu Asn Glu Asp Ala Val Thr Thr Gln Asp
   325 330 335
   Asn Gly Ala Asn Pro Met Asp Leu Trp Ser Ile Asp Glu Ile His Ser
   340 345 350
   Met Ile Gly Gly Val Phe
   355
<210> 3
   <211> 24
   <212> DNA
   <213> Artificial Sequence
<220>
   <223> Primer
<400> 3
   atgtgtggag ctataatatc cgat 24
<210> 4
   <211> 26
   <212> DNA
   <213> Artificial Sequence
<220>
   <223> Primer
<400> 4
   tcagaagact cctccaatca tggaat 26
<210> 5
   <211> 25
   <212> DNA
   <213> Artificial Sequence
<220>
   <223> Primer
<400> 5
   cgttgatgct ggatgtaatg ggtat 25
<210> 6
   <211> 25
   <212> DNA
   <213> Artificial Sequence
<220>
   <223> Primer
<400> 6
   cctgagtcgt tacagcatct tcgtt 25
<210> 7
   <211> 25
   <212> DNA
   <213> Artificial Sequence
<220>
   <223> Primer
<400> 7
   ctaactgcat caagaacaca gagaa 25
<210> 8
   <211> 25
   <212> DNA
   <213> Artificial Sequence
<220>
   <223> Primer
<400> 8
   agatatcaca tcaatccact tgctt 25
<210> 9
   <211> 22
   <212> DNA
   <213> Artificial Sequence
<220>
   <223> Primer
<400> 9
   gcgtggaccg cttgctgcaa ct 22
<210> 10
   <211> 25
   <212> DNA
   <213> Artificial Sequence
<220>
   <223> Primer
<400> 10
   tgttaaatat ctgttctgcg ttggc 25
<210> 11
   <211> 21
   <212> DNA
   <213> Artificial Sequence
<220>
   <223> Primer
<400> 11
   aaccaattca atcgctcaaa c 21